# EUROPEAN PATENT APPLICATION

(11) **EP 3 964 851 A1**
(43) Date of publication of application: **09.03.2022**
(21) Application number: 20194970.8
(22) Date of filing: 08.09.2020
(51) Int. Cl.: G01R 33/34, G01R 33/54

(54) **METHOD FOR ADAPTING A COIL OF AN MR IMAGING DEVICE**

(71) Applicant: Koninklijke Philips N.V., 5656 AG Eindhoven (NL)
(72) Inventor: LEUSSLER, Christoph, 5656 AE Eindhoven (NL); WEISS, Steffen, 5656 AE Eindhoven (NL); VOGTMEIER, Gereon, 5656 AE Eindhoven (NL); JOHNSON, Mark Thomas, 5656 AE Eindhoven (NL); WIRTZ, Daniel, 5656 AE Eindhoven (NL); SISODIA, Rajendra Singh, 5656 AE Eindhoven (NL)
(74) Representative: Philips Intellectual Property & Standards

(57) **Abstract**

A computer-implemented method for autonomously adapting at least one coil of an MR imaging device, the method comprising the steps: providing shape data of a patient (S100);
calculating and generating a control signal for pre-forming the at least one coil of the MR imaging device based on the provided shape data of the patient (S200), wherein the generated control signal is configured for adapting a position of said coil to a target position and/or for adapting a shape of said coil to a target shape; autonomously adapting the position and/or the shape of said coil based on the generated control signal (S300); generating current imaging data of the patient using the adapted coil (S400); evaluating the position and/or shape of said coil based on the generated imaging data and determining an evaluation result (S500).

## Description

### FIELD OF THE INVENTION

The invention relates to a computer-implemented method for autonomously adapting at least one coil of an MR imaging device, a pre-formation apparatus for autonomously adapting at least one coil of an MR imaging device, an MR imaging system for autonomously adapting at least one coil of an MR imaging device, a use of a coil together with such a pre-formation apparatus and a computer program element.

### BACKGROUND OF THE INVENTION

MR imaging devices are well known in the state of the art and widely used as medical imaging devices in hospitals. In comparison to CT scanners MR imaging devices do not require hazardous X-Rays to acquire an image. Instead MR imaging devices use magnetic fields to generate images of parts of the human body (e.g. organs). The area of the human body to be imaged, an excitation coil and a receiver coil are arranged relative to each other for imaging. The arrangement of these imaging means has an influence on a quality of the imaging.

In view of this, it is found that a further need exists to provide a method for arranging imaging means of an MR imaging device.

### SUMMARY OF THE INVENTION

It is therefore an object of the present invention to provide a method for arranging imaging means of MR imaging device, particularly to provide an improved method for arranging imaging means of MR imaging device.

These and other objects, which become apparent upon reading the following description, are solved by the subject-matter of the independent claims. The dependent claims refer to preferred embodiments of the present disclosure.

According to a first aspect of the present invention a computer-implemented method for autonomously adapting at least one coil of an MR imaging device is provided, the method comprising the steps: providing shape data of a patient; calculating and generating a control signal for pre-forming the at least one coil of the MR imaging device based on the provided shape data of the patient, wherein the generated control signal is configured for adapting a position of said coil to a target position and/or for adapting a shape of said coil to a target shape; autonomously adapting the position and/or the shape of said coil based on the generated control signal; generating current imaging data of the patient using the adapted coil; evaluating the position and/or shape of said coil based on the generated imaging data and determining an evaluation result.

In other words, the present invention proposes to pre-form a coil of a MR imaging device based on already existing geometry data of a patient and to use measurement data from the coil when being applied to the patient for checking a position and/or shape of the coil. By using already existing geometry data of the patient pre-forming of the coils may take place outside the bore of a MRI system and therefor increase a productivity of the MRI system. Carrying out the adapting process outside of the bore the MRI system may also be advantageous to explain and/or train the patient or a technician/operator without wasting valuable production time of the MRI system.

The checking of the position and or the shape of the coil does not require additional sensors, instead is carried by using the already existing equipment, namely the coils. The use of the coils for checking/evaluating the position and/or shape may enable an efficient way of adapting and optimizing an arrangement of patient and coils. In case the measurement data discloses an insufficient position and /or shape of the coils further pre-forming of the coils is initialized. Due to an autonomous and continuous process of pre-forming and evaluating /checking the imaging process may be more efficient and the quality of the images may increase. It might further be advantageous to check the autonomous pre-forming process of the coils by evaluating the measurement data of the coils in order to detect position and/or shape errors. Errors might occur due improper geometry data of the patient or errors of actuators of the coils. The autonomous pre-forming process may advantageously lead to a reduced work load of the technician/operator, an increased productivity of the MRI system and an increased image quality of the MRI system.

The term shape data is to be understood broadly and comprises any geometry information of the patient, e.g. curves, areas, coordinates, dimensions of the body and also position/dimensions/content of organs or bones in the body. The term control signal is to be understood broadly and comprises any information for controlling actuators of the MR imaging device. The actuators comprise pneumatic, hydraulic or electro mechanical drives. The actuators change the form and position of the coils of the MR imaging device. The MR imaging device is to be understood broadly and comprises any components used to acquire MR image data. Preferably the MR imaging device comprises an excitation coil and a receiver coil. The position is to be understood broadly and comprises any spatial information of the coil. Preferably the position comprises the three rotational degrees of freedom and three translatory degrees of freedom. It should be noticed in this context that a coil may comprise different areas which have different positions. The term shape is to be understood broadly and comprises any geometry information of the coils, preferably surface dimensions and surface orientations. The term target position and target shape comprise a desired position and a desired shape of the coil. The term imaging data is to be understood broadly and comprises imaging data related to the MR imaging device, wherein the image data is not limited to acquire images inside the bore of MR system. Preferably the imaging data comprises reconstructed image, raw data, low resolution imaging or just real time noise measurement. E.g. the noise of an individual coil and a noise correlation matrix give a measure of how close the coil is located to the patient. This kind of noise measurement can also be applied outside the MRI bore, when the patient is on a patient bridge/bed. The term evaluation result is to be understood broadly and comprises and comprises any comparison of measurement data with reference values, wherein the reference values relate to the desired position and or shape of the coil.

In an embodiment the method further comprises the step autonomously adapting the position and/or the shape of said coil based on a result of said evaluation. With other words the position and/or the shape of the coil are continuously adapted until the target shape/point is reached. This may advantageously increase the quality of medical images. Preferably the step autonomously adapting the position and/or shape of said coil based on a result of said evaluation is carried out as a feedback control.

In an embodiment the shape data of the patient is an MR scan or a CT scan or 3D shape sensor. The shape data of the patient is not limited to before mentioned examples. The shape data of the patient may be provided from a data base or previous medical treatments. The shape data may be transferred into a desired data format for further processing such as generating control signals. The shape data may constructed from different sources, e.g. CT scan and 3D shape sensor. By enabling the use of different shape data sources and/or data formats the production efficiency and applicability of the MR imaging process may increase.

In an embodiment the method further comprises the steps: calculating and generating a control signal for pre-forming at least one mattress and/or at least one dielectric pad used in combination with the MR imaging device based on the provided shape data of the patient, wherein the generated control signal is configured for adapting a position of said mattress and/or dielectric pad to a target position and/or for adapting a shape of said mattress and/or dielectric pad to a target shape; autonomously adapting the position and/or the shape of said mattress and/or dielectric pad based on the generated control signal; generating a current imaging data of the patient; evaluating the position and/or shape of said mattress and/or dielectric pad based on the generated imaging data and determining an evaluation result, autonomously adapting the position and/or the shape of said mattress and/or dielectric pad based on the evaluation result. The term mattress means in the following the mattress on which patient is positioned. The mattress comprises actuators for adapting the position / and or shape of the mattress. By adapting the position or shape of the mattress the position and or shape of the body of the patient may also advantageously adapted. The term dielectric pad comprises simple pouches containing a highly conductive material that are placed between the patient and the receiver coil to reduce dielectric artifacts. The simplest dielectric pads comprise a dilute solution or gel comprising manganese chloride. More complex solid dielectric pads comprise suspensions of metal (primarily barium and calcium), titanates in deuterated or protonated water. The dielectric pads be used separately or be integrated or combined in/with the coil. With other words the method for autonomously adapting at least one coil of an MR imaging device is extended to adapt also the mattress and the dielectric pad. The mattress and the dielectric pad and the coils interact with each other in combination with the patient's body and form an arrangement for MR imaging. Hence, it may be advantageously to adapt and control not only the coil but also the mattress and the dielectric pad. This may increase productivity, efficiency and quality of the imaging process.

In an embodiment the at least one coil is selected from a group of different pre-tensioned coils based on the shape of the patient data. The pre-tensioned coils comprise different degrees of shape flexibility, wherein the different shape flexibility may influence and increase the patient's degree of comfort. The alignment of different pre-tensioned coils to the shape data of the patient may also consider previous treatment data of the patient.

In an embodiment the method further comprises the step storing the target form and position of said coil and/or of said mattress and/or of said dielectric pad. The stored data of the target form and position of the coil, the mattress or the dielectric pad may enable analysis of the history of treatments of the patient and/or an additional back up check of the current determined target form and or position. The stored data of the target form may be used if the patient returns and the exact same examination has to be carried out. In case of further adaptions, the stored data may be used as starting values for the method.

According to a further aspect of the present invention a pre-formation apparatus for autonomously adapting at least one coil of an MR imaging device is provided, comprising: a receiving unit configured to receive shape data of a patient; a calculation unit configured to calculate and generate a control signal for pre-forming the at least one coil of the MR imaging device based on the provided shape data of the patient, wherein the generated control signal is configured for adapting a position of said coil to a target position and/or for adapting a shape of said coil to a target shape; an adapting unit configured to autonomously adapt the position and/or the shape of said coil based on the generated control signal; wherein the calculation unit is configured to analyze a current imaging data of the patient in which the adapted coil is used; and wherein the calculation unit is configured to evaluate the position and/or shape of said coil based on the current imaging data and to determine an evaluation result. The receiving unit, the calculation unit and the adapting unit may be separated hardware units such as a CPU, NC, PC or virtual units which were implemented on a one or more hardware unit such as e.g. CPU.

In an embodiment a pre-formation apparatus is provided, wherein the adapting unit is further configured to autonomously adapt the position and/or the shape of said coil based on the evaluation result. With other words the position and/or the shape of the coil are continuously adapted until the target shape/point is reached. This may advantageously increase the quality of medical images. Preferably the step autonomously adapting the position and/or shape of said coil based on a result of said evaluation is carried out as a feedback control.

According to a further aspect of the present invention an MR imaging system for autonomously adapting at least one coil of an MR imaging device is provided, comprising: a pre-formation apparatus as described above; the MR imaging system configured to image at least a part of a body of the patient. The pre-formation apparatus and the MR imaging system show advantageous synergetic effects concerning production efficiency and the quality of the acquired images.

In an embodiment the system further comprises a display configured to display the patient and the at least one coil and or mattress and/or electrical pad. The display may serve an operator as visual checkup possibility for possible errors, which may be required due to the autonomous adapting process. The display may increase the safety of the MR-imaging system and the productivity of the MR imaging system. The patient and the at least one coil and or mattress and/or electrical pad may also be visualized via augmented reality. The operator may be enabled to take over control via an interface to manually adapt the position and or shape of the coil, mattress or dielectric pad. The patient may also adapt the position and or shape of the coil, mattress or dielectric pad himself by instructions of the operator.

In an embodiment the system further comprises a determination unit configured to determine a level of adaptability of the at least one coil and to provide the level of adaptability to an operator. The coils show different capabilities for being adapted respectively changed in shape and/or position (i.e. level of adaptability). The determination unit may improve the selection of the coils for an operator due to additional information of the coils, namely the level of adaptability. Hence, the selection process and the overall imaging process may be improved. A visualization of the level of adaptability may be based on a traffic color coding mechanism. The level of adaptability may relate to a pain effecting the patient, e.g. if the mattress has to be changed in a very high degree.

In an embodiment the system further comprises: a training unit configured to provide a training program for a patient lying on a bed outside a bore of the MR system and/or for an operator; and wherein the training program comprises: providing shape data of a patient; calculating and generating a control signal for pre-forming the at least one coil of the MR imaging device based on the provided shape data of the patient, wherein the generated control signal is configured for adapting a position of said coil to a target position and/or for adapting a shape of said coil to a target shape; autonomously adapting the position and/or the shape of said coil based on the generated control signal; providing feedback information to the patient and/or the operator. The training unit may be a separated hardware unit such as a CPU, NC, PC or a virtual unit which is implemented on a one or more hardware units such as e.g. CPU. The training program may help a patient to reduce possible fears of MR imaging process. The training program may help an operator to train the MR imaging system and to increase productivity of MR imaging system. Possible results from the training program may be reused in a current MR imaging process, e.g. positions/shapes of coils, mattress, dielectric pads, and selection of pre-tensioned coils. The training program may further comprise a chat-bot communicating via speech, visual signal or acoustic signal to the patient.

In an embodiment the system is provided, wherein the feedback information comprises light signal, acoustic signal, actuators and/or speech. The feedback information helps to guide the patient and to gain his confidence. During the autonomously adapting process of the coil, mattress or dielectric pad the feedback information may also be presented to the patient e.g. via screen or augmented reality googles in order to view the operation going around him. This may reduce anxiety.

In an embodiment the system is provided, wherein the coils, mattresses or dielectric pads automatically flatten themselves, when removed from the patient after use. In this content the system may preferably give advice on if the coils, mattresses or pads will have to be removed for the next scan and in case if so, guide the patient to do it himself. In this content the system may preferably give advice on to initial place the coil, mattress or dielectric pads before the first scan. In sum this may increase the imaging productivity of the MRI system.

A further aspect of the present invention relates to a use of a coil together with a pre-formation apparatus.

A last aspect of the present invention relates to a computer program element, which when executed by a processor is configured to carry out a method for autonomously adapting at least one coil of an MR imaging device.

### BRIEF DESCRIPTION OF THE DRAWINGS

Fig 1 shows a schematic view of a MR imaging device; and
Fig. 2 shows a flow chart of a method for autonomously adapting at least one coil of an MR imaging device according to the present disclosure.

### DETAILED DESCRIPTION OF EMBODIMENTS

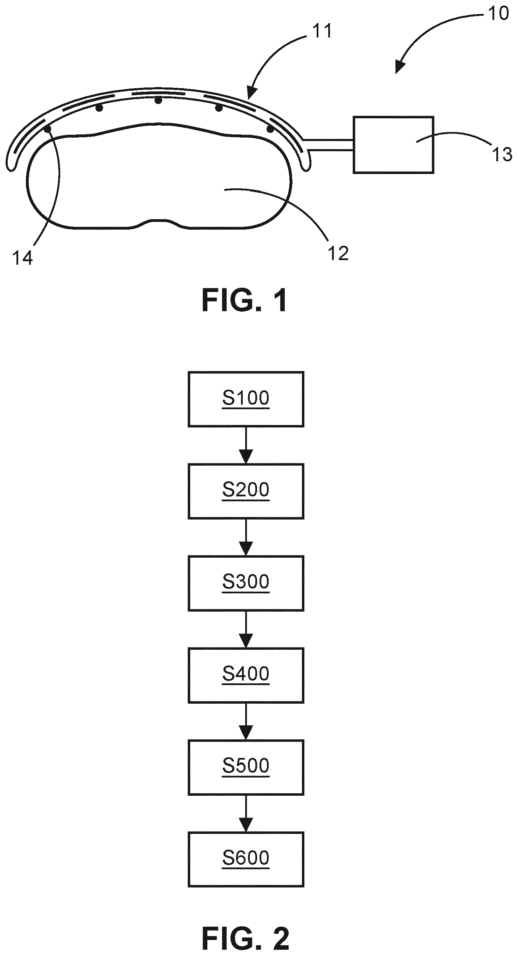

Fig. 1 shows a schematic view of a MR imaging device. The MR imaging device 10 comprises a coil 11 for imaging a body part 12 of a patient. The coil is connected to actuator 13, in the present case pneumatic drive 13. The pneumatic drive is configured to change the position and or shape of the coil 11. The pneumatic drive 13 changes the position and or shape of the coil 11 based on a control signal. The control signal is determined by a calculation unit (not shown) based on the shape data of the patient. The coil 11 comprises further sensors 14 for measuring additionally the homogeneity of the air gaps between the body part 12 and the coil 11. The coil may send control commands and triggers via an interface to the MRI system (not shown) (e.g. patient ready for start, breathing trigger, etc.). By measuring variations in signal of coils and/or sensors, moving coil or dielectric pads (not shown) may be discriminated from the stationary background allowing to measure stress or pain by detecting an individual's breathing, heart rate, skin conductance (GSR) and movement.

Fig. 2 shows a flow chart of a method for autonomously adapting at least one coil of an MR imaging device according to the present disclosure.

When a patient is scheduled for a clinical imaging-procedure the method for autonomously adapting at least one coil of an MR imaging device start. In step S 100 shape data of a patient is provided. The shape data may be stored in a hospital IT system and comprise CT data or MRI data from previous medical treatments. Based on the shape data and medical treatment, coils, mattress and/or dielectric pads are selected. Afterwards the coil, mattress or dielectric pads and the patient to be imaged are initially placed on a patient bed outside the bore and/or in the bore before the first scan starts. In step S200 a control signal for pre-forming the at least one coil of the MR imaging device based on the provided shape data of the patient is calculated and generated, wherein the generated control signal is configured for adapting a position of said coil to a target position and/or for adapting a shape of said coil to a target shape. The control signal is derived from the shape data of the patient and takes also into account the upcoming medical treatment. In step S300 the position and/or the shape of the coil is autonomously adapted based on the generated control signal, wherein the patient lies on a patient bed and e.g. the coil is above arranged. The adaption is carried by e.g. pneumatic drives. In step S400 current imaging data of the patient using the adapted coil is generated. The imaging data may be MRI imaging data generated by the MRI system. In step S500 the position and/or shape of said coil based on the generated imaging data is evaluated and an evaluation result is determined. In step S600 the position and/or the shape of said coil based on a result of said evaluation is further autonomously adapted. The process continuous until a sufficient image quality is reached.

### LIST OF REFERENCE SIGNS:

- 10: MR imaging device
- 11: coil
- 12: body part
- 13: pneumatic drive
- 14: sensor

## Claims

1. A computer-implemented method for autonomously adapting at least one coil of an MR imaging device, the method comprising the steps:
providing shape data of a patient (S100);
calculating and generating a control signal for pre-forming the at least one coil of the MR imaging device based on the provided shape data of the patient (S200),
wherein the generated control signal is configured for adapting a position of said coil to a target position and/or for adapting a shape of said coil to a target shape;
autonomously adapting the position and/or the shape of said coil based on the generated control signal (S300);
generating current imaging data of the patient using the adapted coil (S400);
evaluating the position and/or shape of said coil based on the generated imaging data and determining an evaluation result (S500).

2. The method according to claim 1, further comprising the step autonomously adapting the position and/or the shape of said coil based on a result of said evaluation (S600).

3. The method according to claim 1 or 2, wherein the shape data of the patient is an MR scan or a CT scan or 3D shape sensor.

4. The method according to one of the preceding claims, further comprising the steps:
calculating and generating a control signal for pre-forming at least one mattress and/or at least one dielectric pad used in combination with the MR imaging device based on the provided shape data of the patient,
wherein the generated control signal is configured for adapting a position of said mattress and/or dielectric pad to a target position and/or for adapting a shape of said mattress and/or dielectric pad to a target shape;
autonomously adapting the position and/or the shape of said mattress and/or dielectric pad based on the generated control signal;
generating current imaging data of the patient;
evaluating the position and/or shape of said mattress and/or dielectric pad based on the generated imaging data and determining an evaluation result,
autonomously adapting the position and/or the shape of said mattress and/or dielectric pad based on the evaluation result.

5. The method according to one of the preceding claims, wherein the at least one coil is selected from a group of different pre-tensioned coils based on the shape of the patient data.

6. The method according to claim 4, further comprising the step:
storing the target form and position of said coil and/or of said mattress and/or of said dielectric pad.

7. A pre-formation apparatus for autonomously adapting at least one coil of an MR imaging device, comprising:
a receiving unit configured to receive shape data of a patient;
a calculation unit configured to calculate and generate a control signal for pre-forming the at least one coil of the MR imaging device based on the provided shape data of the patient,
wherein the generated control signal is configured for adapting a position of said coil to a target position and/or for adapting a shape of said coil to a target shape;
an adapting unit configured to autonomously adapt the position and/or the shape of said coil based on the generated control signal;
wherein the calculation unit is configured to analyze current imaging data of the patient in which the adapted coil is used;
and wherein the calculation unit is configured to evaluate the position and/or shape of said coil based on the current imaging data and to determine an evaluation result.

8. The pre-formation apparatus according to claim 7, wherein
the adapting unit is further configured to autonomously adapt the position and/or the shape of said coil based on the evaluation result.

9. An MR imaging system for autonomously adapting at least one coil of an MR imaging device, comprising:
a pre-formation apparatus according to claim 7 or 8;
the MR imaging system configured to image at least a part of a body of the patient.

10. The system according to claim 9 further comprising a display configured to display the patient and the at least one coil and or mattress and/or electrical pad.

11. The system according to claim 9 or 10, further comprising a determination unit configured to determine a level of adaptability of the at least one coil and to provide the level of adaptability to a operator.

12. The system according to one of the claims 9 to 11, further comprising:
a training unit configured to provide a training program for a patient lying on a bet outside a bore of the MR system and/or for an operator; and
wherein the training program comprises:
providing shape data of a patient;
calculating and generating a control signal for pre-forming the at least one coil of the MR imaging device based on the provided shape data of the patient,
wherein the generated control signal is configured for adapting a position of said coil to a target position and/or for adapting a shape of said coil to a target shape; autonomously adapting the position and/or the shape of said coil based on the generated control signal;
providing feedback information to the patient and/or the operator.

13. The system according to claim 12, wherein the feedback information comprises light signal, acoustic signal, actuators and/or speech.

14. Use of a coil together with a pre-formation apparatus according to one of the claims 7 to 9.

15. A computer program element, which when executed by a processor is configured to carry out a method according to one of the claims 1 to 6.
